# FASCICULE DE BREVET EUROPEEN

(11) **EP 2 070 516 B1**
(45) Date de publication et mention de la délivrance du brevet: **14.08.2013**
(21) Numéro de dépôt: 08170557.6
(22) Date de dépôt: 03.12.2008
(51) Int. Cl.: A61K 8/73, A61K 8/891, A61K 8/81, A61K 8/893, A61K 8/898, A61Q 5/06

(54) **Procédé de coloration des cheveux à partir d'une composition comprenant un polymère filmogène hydrophobe, un pigment et un solvant volatil**
Haarfärbeverfahren mit einer Zusammensetzung enthaltend ein filmbildendes Polymer, ein Pigment und ein flüchtiges Lösungsmittel
Process for dyeing the hair using a composition comprising a hydrophobic film-forming polymer, a pigment and a volatile solvent

(30) Priorité: 13.12.2007 FR 0759814
(43) Date de publication de la demande: 17.06.2009
(73) Titulaire: L'Oréal, 75008 Paris (FR)
(72) Inventeur: Brun, Gaëlle, 75015 Paris (FR)
(74) Mandataire: Fevrier, Murielle Françoise E.

(56) Documents cités:
- WO-A-2006/057071
- FR-A- 2 853 528
- FR-A- 2 899 804
- US-A- 6 073 635

## Description

La présente invention a pour objet un procédé de coloration des cheveux, à partir d'un polymère filmogène hydrophobe et d'un pigment.

Dans le domaine de la coloration des fibres kératiniques, il est déjà connu de colorer des fibres kératiniques par différentes techniques à partir de colorants directs ou de pigments pour des colorations non permanentes ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Pour être visible sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou de persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR2741530, qui préconise l'utilisation pour la coloration temporaire des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

Il est par ailleurs connu d'effectuer des gainages colorés des cheveux à partir d'une composition comprenant un monmère électrophile de type cyanoacrylate, et un pigment notamment dans le document EP1649898. Une telle composition permet d'obtenir des cheveux parfaitement gainés et non gras. Cependant, le gainage obtenu ne donne pas une totale satisfaction face aux agents extérieurs tels que le lavage et la transpiration. Par ailleurs le gainage obtenu est sensible aux corps gras tels que le sébum.

Ainsi, le but de la présente invention est de mettre au point un procédé de coloration des fibres kératiniques telles que les cheveux qui permet d'obtenir des gainages colorés rémanents aux shampoings et aux diverses agressions que peuvent subir les cheveux sans dégradation des fibres kératiniques et tout en conservant des cheveux parfaitement individualisés.

Ce but est atteint avec la présente invention qui a pour objet un procédé de coloration des cheveux comprenant l'application sur les cheveux d'une composition comprenant un ou plusieurs polymères filmogènes hydrophobes choisis parmi les polymères ou copolymères à base de polyuréthane, de polyacrylate, les copolymères d'ester/vinyliques, les résines de silicone, les silicones polyurée/polyuréthane, les copolymères à base de résine de silicone et de diméthiconol, au moins un pigment et un ou plusieurs solvants volatils, suivie d'une étape de chauffage à une température supérieure à 40°C des cheveux recouverts de la composition.

Par l'utilisation d'un tel procédé, on obtient sur les fibres kératiniques des gainages colorés permettant d'obtenir une coloration visible sur tous types de cheveux de façon rémanente aux shampooings tout en préservant les qualités physiques de la fibre kératiniques. Un tel gainage est en particulier résistant aux agressions extérieures que peuvent subir les cheveux telles que le brushing et la transpiration. Il permet en particulier d'obtenir un dépôt lisse et homogène. Par ailleurs, on a constaté de façon surprenante que les cheveux restaient parfaitement individualisés, pouvaient être coiffés sans problème et que les propriétés de coiffant apportées à la fibre étaient rémanentes aux shampooings.

On entend par cheveux individualisés des cheveux qui après application de la composition et séchage ne sont pas collés (ou sont tous séparés les uns des autres) entre eux et ne forment donc pas des amas de cheveux, le gainage étant formé autour de pratiquement chaque cheveu.

Selon l'invention, la composition contient un polymère filmogène hydrophobe et un pigment dans un solvant volatil.

Par "polymère", on entend au sens de l'invention un composé correspondant à la répétition d'un ou plusieurs motifs (ces motifs étant issus de composés appelés monomères). Ce ou ces motifs sont répétés au moins deux fois et de préférence au moins 3 fois.

Par polymère hydrophobe, on entend un polymère ayant une solubilité dans l'eau à 25°C inférieure à 1 % en poids.

Par polymère "filmogène", on entend un polymère apte à former à lui seul ou en présence d'un agent auxiliaire de filmification, un film macroscopiquement continu sur un support, notamment sur les matières kératiniques, et de préférence un film cohésif.

Dans un mode de réalisation, le polymère organique filmogène hydrophobe est au moins un polymère choisi parmi le groupe comprenant :
- les polymères filmogènes solubles dans un milieu solvant organique, en particulier les polymères liposolubles; cela signifie que le polymère est soluble ou miscible dans le milieu organique et va former une seule phase homogène lorsqu'il sera incorporé dans le milieu.
- les polymères filmogènes dispersibles dans un milieu solvant organique, cela signifie que le polymère forme une phase insoluble dans le milieu organique, le polymère restant stable et/ou compatible une fois incorporé dans ce milieu. En particulier de tels polymères peuvent se présenter sous la forme de dispersions non aqueuses de particules de polymères, de préférence des dispersions dans les huiles siliconées ou hydrocarbonées; dans un mode de réalisation, les dispersions non aqueuse de polymère comprennent des particules polymères stabilisées sur leur surface par au moins un agent stabilisant ; ces dispersions non aqueuses sont souvent appelées « NAD (non-aqueous dispersions) ».
- les polymères filmogènes sous forme de dispersions aqueuses de particules de polymère, cela signifie que le polymère forme une phase insoluble dans l'eau, le polymère restant stable et/ou compatible une fois incorporé dans l'eau, les particules de polymère pouvant être stabilisées sur leur surface par au moins un agent stabilisant. Ces particules de polymère sont souvent appelées « latex» ;
dans ce cas, la composition doit comprendre une phase aqueuse.

Parmi l'ensemble des polymères filmogènes hydrophobes utilisables dans la composition de la présente invention, on peut citer les polymères synthétiques, de type radicalaire ou de type polycondensat, les polymères d'origine naturelle et leurs mélanges. Parmi ceux-ci on compte les polymères ou les copolymères de l'invention, à savoir ceux à base de polyuréthane, de polyacrylate, les copolymères d'ester vinyliques, les résines de silicones, les silicones polyurée/ polyuréthane, les copolymères à base de résine de silicone et de diméthiconol; ils sont spécifiés dans la suite. Les polymères filmogènes hydrophobes décrits dans la suite, autres que ceux de l'invention, peuvent être présents de façon optionnelle.

Parmi les polymères filmogènes hydrophobes, on compte les polymères filmogènes décrits dans la demande WO 04/028487 et:
a) Les homopolymères et les copolymères des oléfines ; des cyclooléfines; du butadiène ; de l'isoprène ; du styrène ; des éthers, des esters ou amides vinyliques ; des esters ou amides de l'acide (méth)acrylique contenant un groupement alkyle en C₁-C₂₀, linéaire, ramifié ou cyclique, un groupement aryle en C₆-C₁₀ ou un groupement hydroxyalkyle en C₂-C₆.

De tels homopolymères et copolymères peuvent être obtenus à partir de monomères choisis parmi le groupe constitué par le (méth)acrylate d'isooctyle, le (méth)acrylate d'iso-nonyle, le (méth)acrylate de 2-éthylhexyle, le (méth)-acrylate de lauryle, le (méth)acrylate d'isopentyle, le (méth)acrylate de n-butyle, le (méth)acrylate d'iso-butyle, le (méth)acrylate d'éthyle, le (méth)acrylate de méthyle, le (méth)acrylate de tertio-butyle, le (méth)acrylate de tridécyle, le (méth)acrylate de stéaryle, le (méth)acrylate d'hydroxyéthyle, le (méth)acrylate de 2-hydroxypropyle, l'acrylate de benzyle ou de phényle ou des mélanges de ceux-ci. Comme amides des monomères acides, on peut citer les (méth)acrylamides, et notamment les N-alkyl (méth)acrylamides, en particulier d'alkyle en C₂-C₁₂ tels que le N-éthyl acrylamide, le N-t-butyl acrylamide, le N-octyl acrylamide; les N- dialkyl (C₁-C₄) (méth)acrylamides, les (méth)acrylates de perfluoroalkyle. Les polymères ci-dessus peuvent également contenir à titre de monomères de faibles quantités d'un acide carboxylique ou sulfonique insaturés tels que les acides acryliques, méthacryliques, AMPS, à condition que le caractère global du polymère demeure hydrophobe.

Comme autres monomères vinyliques utilisables, on peut encore citer :
- la N-vinylpyrrolidone, la vinylcaprolactame, les vinyl N-alkyl(C₁-C₆) pyrroles, les vinyl-oxazoles, les vinyl-thiazoles, les vinylpyrimidines, les vinylimidazoles,
- les oléfines tels que l'éthylène, le propylène, les butènes, l'isoprène, les butadiènes.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

On citera, par exemple, le copolymère d'acrylate d'alkyle/acrylate de cycloalkyle commercialisé par PHOENIX CHEM. sous la dénomination GIOVAREZ AC-5099 ML, le copolymère acrylates/C₁₂-₂₂ alkyl methacrylate commercialisé par ROHM AND HAAS sous la dénomination SOLTEX OPT et les copolymères de vinylpyrrolidone, tels que les copolymères d'un alkène en C₂ à C₃₀, tel qu'en C₃ à C₂₂, et des associations de ceux-ci, peuvent être utilisés. Comme exemples de copolymères de VP pouvant être utilisés dans l'invention, on peut aussiciter le copolymère de VP/laurate de vinyle, de VP/stéarate de vinyle, la polyvinylpyrrolidone (PVP) butylée, de VP/hexadécène commercialisé par ISP sous la dénomination Ganex V216, de VP/eicosène commercialisé par ISP sous la dénomination Ganex V220, de VP/triacontène ou de VP/acide acrylique/méthacrylate de lauryle. On peut également citer les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH ainsi que les copolymères dont la dénomination CTFA est Acrylates/octylacrylamide copolymer, tels que les produits vendus sous la dénomination DERMACRYL® LT ou DERMACRYL® 79 par la société NATIONAL STARCH.

Comme polymères particuliers, on peut citer :
i) les polymères portant des groupements fluorés appartenant à l'une des classes décrites dans le texte ci-dessus, en particulier les Fomblin décrits dans le brevet US 5948393, les copolymères de (méth)acrylate d'alkyle/(méth)acrylate de perfluoroalkyle décrits dans les brevets EP 0815836 et US 5849318.
ii) les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, le polymère filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination «Luvitol HSB» par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés.

On peut citer par exemple le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel MD 970 et le Versagel MD 960 de chez Penreco (mélange de polymère en étoile et de polymère tribloc dans l'isododécane).

Des copolymères de styrène-méthacrylate peuvent également être utilisés tels que les polymères commercialisés sous les référence OS 129880, OS 129881 et OS 84383 de chez Lubrizol (copolymère de styrène-méthacrylate).

Dans un mode de réalisation, le polymère filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui choisi parmi les esters vinyliques (différent de l'ester vinylique déjà présent), les α-oléfines (ayant de 8 à 28 atomes de carbone), les alkylvinyléthers (dont le groupe alkyl comporte de 2 à 18 atomes de carbone), ou les esters allyliques ou méthallyliques (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2% de divinyl benzène, diméthyl propionate de vinyle/laurate de vinyle, réticulé avec 0,2% de divinyl benzène, acétate de vinyle/octadécyl vinyl éther, réticulé avec 0,2% de tétraallyloxyéthane, acétate de vinyle/stéarate d'allyle, réticulé avec 0,2% de divinyl benzène, acétate de vinyle/octadécène-1 réticulé avec 0,2% de divinyl benzène et propionate d'allyle/stéarate d'allyle réticulé avec 0,2% de divinyl benzène.
iii) les polyalcènes, les copolymères d'alcènes en C₂-C₂₀, en particulier le polybutène.
iv) les polymères d'origine naturelle, éventuellement modifiés, pouvant être choisis parmi la résine de shellac, la gomme de sandaraque, les dammars, les élémis, les copals. les polysaccharides comprenant des chaînes latérales alkyl (éther ou ester), en particulier les alkylcelluloses ayant un radical alkyle en C₁ à C₈ saturé ou insaturé, linéaire ou ramifié, tel que l'éthylcellulose et la propylcellulose.

Le polymère filmogène d'origine naturelle peut être choisi en particulier parmi les polymères cellulosiques tels que la nitrocellulose, l'acétate de cellulose, l'acétobutyrate de cellulose, l'acétopropionate de cellulose, On peut citer par exemple l'éthyl cellulose commercialisée par Aqualon sous la référence Aqualon Ethylcellulose N200, l'acétobutyrate cellulose commercialisée par Eastman Chemical sous la référence CAB-381-0.5, les acéto-propionate de cellulose commercialisée par Eastman Chemical sous les références CAP-482-20 et CAP-504-0.2.

### v) les polycondensats

Parmi les polycondensats, on peut citer les polyuréthanes non ioniques, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino).

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques.

Les polyesteramides peuvent être obtenus de manière analogue aux polyesters, au moyen de la polycondensation de diacides avec des aminoalcools. Les polyamides peuvent être obtenus de manière analogue aux polyesters, au moyen de la polycondensation de diacides avec des diamines.

A titre de polyester particulier, on peut citer les polyesters aliphatiques ayant des chaînes latérales alkyle en C₄-₅₀ ou bien les polyesters résultant de la condensation de dimères d'acides gras, voire les polyesters comprenant un segment siliconé sous la forme d'une séquence, greffon ou groupement terminal, tel que défini dans la demande de brevet FR0113920.

### b) Composés siliconés.

Le polymère filmogène hydrophobe peut aussi être un polymère comprenant au moins une partie siliconée.

Dans ce qui suit, on entend désigner par silicone ou polysiloxane, en conformité avec l'acception générale, tous les polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Comme polymère filmogène hydrophobe comprenant au moins une partie siliconée on peut notamment citer :

### i) les résines de silicone, généralement solubles ou gonflables dans les huiles de silicone.

Ces résines sont des polymères de polyorganosiloxanes réticulés.
- La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.
- La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2}.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :
- les siloxysilicates qui peuvent être des triméthylsiloxysilicate de formule [(CH₃)₃XSiXO]ₓX(SiO_{4/2})_{y} (unités MQ) dans laquelle x et y sont des entiers allant de 50 à 80,
- les polysilesquioxanes de formule (CH₃SiO_{3/2})_{.x} (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polyméthylsilsesquioxanes sont décrits dans le document US 5,246,694.

A titre d'exemples de résines polyméthylsilsesquioxanes commercialement disponibles, on peut citer celles qui sont commercialisés :
- par la société Wacker sous la référence Resin MK tels que la Belsil PMS MK :
   polymère comprenant des unités répétitives CH₃SiO_{3/2} (unités T), pouvant aussi
   comprendre jusqu'à 1 % en poids d'unités (CH₃)₂SiO_{2/2} (unités D) et présentant un poids moléculaire moyen d'environ 10000,
- par la société SHIN-ETSU sous les références KR-220L qui sont composés d'unités T de formule CH₃SiO_{3/2} et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88% d'unités T et 12% d'unités dimethyl D et ont des groupes terminaux Si-OH.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.
ii) Les polyamides siliconés du type polyorganosiloxane tels que ceux décrits dans les documents US A 5874069, US A 5919441, US A 6051216 et US A 5981680.
iv) Les composés siliconés greffés

La composition de l'invention peut aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707,EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Le copolymère à squelette organique non-siliconé greffé par des monomères contenant un polysiloxane peut par exemple avoir la structure suivante :

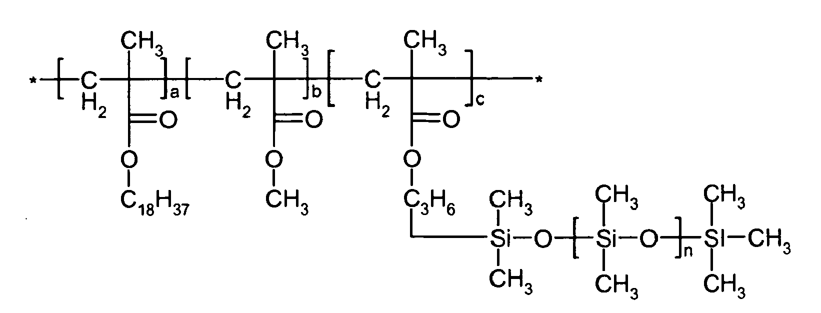

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

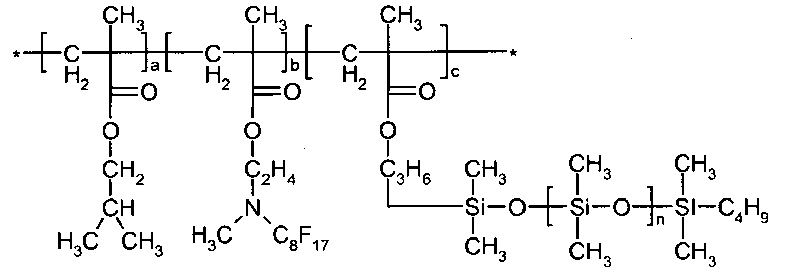

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Comme composé siliconé greffé, on peut également citer le copolymère d'isobutylmethacrylate/bis-hydroxypropyl dimethicone acrylate commercialisé par Grant Industrie sous le nom Granacrysil BMAS.

Selon la présente invention, le ou les polymères siliconés greffés, à squelette polysiloxanique greffé par des monomères organiques non siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Des exemples de polymères siliconés correspondant à la définition sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl/methyl siloxane à groupements propyl thio-3 acrylate de methyle/methacrylate de methyle/acide methacrylique ou Polysilicone-8 commercialisé sous le nom VS80 par la société 3M.

D'autres exemples de polymères siliconés sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl/méthyl siloxane à groupements propyl thio-3-acrylate de méthyle/méthacrylate de méthyle/acide méthacrylique.

### v) Les silicone polyurée/uréthane

Le copolymère de l'invention peut comprendre en plus du polysiloxane/polyurée d'autres blocs de motifs différents. On citera en particulier les terpolymères blocs polysiloxane/polyurée/polyuréthane.

Selon une variante, le copolymère contient uniquement un ou plusieurs blocs siloxane et un ou plusieurs blocs polyurée.

Selon l'invention, le copolymère peut répondre à la formule générale (I) : 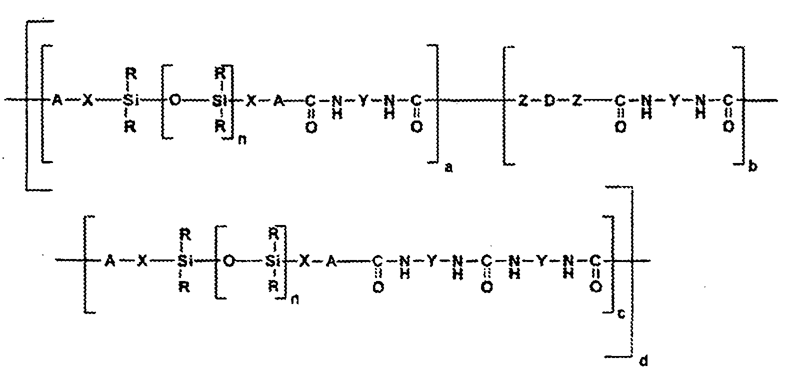 dans laquelle
R représente un radical hydrocarboné monovalent, le cas échéant substitué par le fluor ou le chlore, ayant 1 à 20 atomes de carbone,
X représente un radical alkylène ayant 1 à 20 atomes de carbone, dans lequel des unités méthylène non voisines peuvent être remplacées par des radicaux -O-,
A représente un atome d'oxygène ou un radical amino -NR'-,
Z représente un atome d'oxygène ou un radical amino -NR'-,
R' représente hydrogène ou un radical alkyle ayant 1 à 10 atomes de carbone,
Y représente un radical hydrocarboné bivalent, le cas échéant substitué par le fluor ou le chlore, ayant 1 à 20 atomes de carbone,
D représente un radical alkylène, le cas échéant substitué par fluor, chlore, alkyle en C₁-C₆ ou ester d'alkyle en C₁-C₆, ayant 1 à 700 atomes de carbone, dans lequel des unités méthylène non voisines peuvent être remplacées par des radicaux -O-, -COO-, -OCO- ou -OCOO-,
n est un nombre allant de 1 à 4000,
a est un nombre d'au moins 1,
b est un nombre allant de 0 à 40,
c est un nombre allant de 0 à 30, et
d est un nombre supérieur à 0.
à la condition que A représente dans au moins un des motifs (a) un radical NH

De préférence, R représente un radical hydrocarboné monovalent avec 1 à 6 atomes de carbone par exemple méthyle, éthyle, vinyle et phényle. Selon un mode de réalisation particulier, R est un radical alkyle non substitué.

De préférence, X représente un radical alkylène avec 2 à 10 atomes de carbone. De préférence, le radical alkylène X n'est pas interrompu.

Selon un mode de réalisation particulier, le groupement A dans tous les motifs (b) et (c), lorsqu'ils sont présents, représente NH.

Selon un mode de réalisation particulièrement préféré, tous les groupements A représentent un radical NH.

De préférence, Z représente un atome d'oxygène ou un radical NH.

De préférence, Y représente un radical hydrocarboné comprenant de 3 à 13 atomes de carbone, qui est de préférence, non substitué. De préférence, Y représente un radical aralkylène, alkylène, linéaire ou cyclique.

De préférence, D représente un radical alkylène avec au moins 2, en particulier au moins 4 atomes de carbone et au maximum 12 atomes de carbone.

De préférence également, D représente un radical polyoxyalkylène, en particulier un radical polyoxyéthylène ou polyoxypropylène avec au moins 20, en particulier au moins 100 atomes de carbone et au maximum 800, en particulier au maximum 200 atomes de carbone.

De préférence, le radical D n'est pas substitué.

De préférence, n représente un nombre d'au moins 3, en particulier au moins 25 et de préférence, au maximum 800, en particulier au maximum 400, de manière particulièrement préférée, au maximum 250.

De préférence, a représente un nombre de plus de 50.

Lorsque b est différent de 0, b représente de préférence, un nombre d'au maximum 50, en particulier au maximum 25.

De préférence, c représente un nombre d'au maximum 10, en particulier au maximum 5.

Les copolymères de l'invention peuvent être obtenus selon les procédés de polymérisation décrits dans la demande de brevet US 2004/0254325 ou la demande WO 03/014194.

Selon un autre mode de réalisation, le copolymère est un copolymère polysiloxane/polyurée non ionique, c'est-à-dire qu'il ne contient pas de groupement ionisé ou ionisable.

A titre d'exemple de copolymère, on peut citer le copolymère diméthylpolysiloxane/urée, de nom INCI polyureadimethicone.

Un tel polymère peut être obtenu notamment par copolymérisation d'un alpha,oméga-aminosilicone avec un di-isocyanate. Des polymères répondant à ces caractéristiques sont par exemple les produits commercialisés sous la référence Wacker-Belsil ® UD 60 Wacker-Belsil ® UD 80, Wacker-Belsil ® UD 140 et Wacker-Belsil ® UD 200 par la société Wacker.

### vi) Les copolymères à base de résine de silicone et de silicone fluide

Ces copolymères siliconés sont obtenus par réaction d'une résine de silicone et d'une silicone fluide.

De tels copolymères sont décrits par exemple dans « Silicone Pressure Sensitive Adhesive », Sobieski and Tangney, Handbook of Pressure Sensitive Adhesive Technology (D. Satas Ed.), Von Nostrand Reinhold, New York.

Dans le copolymère, la résine de silicone est présente à un taux compris entre 45 et 75% (par rapport à la masse totale de silicone) et la silicone fluide est présente à un taux compris entre 25 et 55%, avec la somme des pourcentages de résine de silicone et de silicone fluide qui est égal à 100. De préférence, la résine de silicone est présente à un taux compris entre 55 et 65% (par rapport à la masse totale de silicone) et la silicone fluide est présente à un taux compris entre 35 et 45%, avec la somme des pourcentages de résine de silicone et de silicone fluide qui est égal à 100.

De préférence, la résine de silicone selon l'invention est le produit de condensation de groupements SiO₂ et de groupements R₃(SiO)1/2 (triorganosilyl) pour lequel chaque groupement R est indépendamment sélectionné parmi les radicaux méthyle, éthyle, propyle ou vinyl et pour lequel le rapport entre les fonctions SiO₂ et les fonctions R₃(SiO)1/2 de la résine de silicone va de 0.6 à 0.9. Des groupements triorganosilyl utilisables pour former la résine de silicone peuvent être des unités triméthylsilyl, triéthylsilyl, méthylméthylproprylsilyl, diméthylvinylsilyl et des mélanges de celles-ci. Le groupement triméthylsilyl est le préféré dans le cadre de l'invention.

De préférence, la silicone fluide selon l'invention est une diorganopolysiloxane aux fonctions OH terminales ayant une viscosité comprise entre 100 et 100.000 cst à 25°C pour laquelle les substituants du diorganopolysiloxane sont indépendamment choisis parmi les radicaux méthyle, éthyle, propyle ou vinyl. Les diorganosiloxances sont de préférence des polymères linéaires. Des exemples de diorganopolysiloxane peuvent être, de manière non limitative, une polydiméthylsiloxane, une éthylméthylpolysiloxane, un copolymère de diméthylsiloxane et de méthylvinylsiloxane, et des mélanges de tels polymères ou copolymères ayant des extrémités OH. Le diorganopolysiloxane préféré est une polydiméthylsiloxane.

Des exemples de synthèse d'un tel copolymère sont par exemple décrits dans le brevet US5162410 ou dans le brevet CA711756.

Les copolymères préférés selon l'invention sont commercialisés par Dow Corning sous la référence BIO-PSA®, ces BIO-PSA® pouvant être sous deux formes, standard ou amine compatible, et étant fournis dans différents solvants avec plusieurs ratio résine de silicone/silicone fluide. On peut notamment citer les grades 7-4400, 7-4500, 7-4600. Le BIO-PSA® particulièrement préféré selon l'invention est le grade 7-4400..

### vii) Silicones réactives

Le polymère filmogène hydrophe peut être choisi parmi les polymères obtenus à partir de composés siliconés X et Y susceptibles de réagir entre eux au moment de l'application pour former le polymère filmogène hydrophobe. Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO- , - COO- , -OH , -NH₂ , -NH-,-NR-, -SO₃H, -SO₃⁻, -OCH₂CH₂-, -O-CH₂CH₂CH₂-, -O-CH₂CH(CH₃)-, -NR₃⁺, -SH, -NO₂, I, Cl, Br, -CN, -PO₄ ³⁻, -CONH- , -CONR-, -CONH₂, -CSNH-, -SO₂- , -SO-, -SO₂NH-, -NHCO- , -NHSO₂- , -NHCOO-, -OCONH-, -NHCSO- et -OCSNH-, R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique avantageusement en présence d'un catalyseur, par une réaction d'hydrosilylation ou une réaction de condensation, ou une réaction de réticulation en présence d'un peroxyde.

A titre d'exemple d'une combinaison de composés X et Y réagissant par hydrosilylation, on peut citer les références suivantes proposée par la société Dow Corning : DC 7-9800 Soft Skin Adhesive parts A & B, ainsi que les mélanges A et B suivants préparés par Dow Corning :

| **MELANGE A :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

| **MELANGE B :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

Les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, en particulier l'eau apportée par une source extérieure, par exemple par humidification préalable des cheveux (par exemple par un brumisateur).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

De tels polymères sont notamment décrits dans les documents US3175993, US4772675, US4871827, US4888380, US4898910, US4906719 et US4962174, WO 01/96450.

A titre d'exemple d'une combinaison de composés X et Y porteurs de groupements alcoxysilanes et réagissant par condensation, on peut citer les mélanges A' et B4 suivants préparés par la société Dow Corning :

| **Mélange A' :** | | | |
|---|---|---|---|
| **Ingrédient (Nom INCI)** | **N°CAS** | **Teneurs (%)** | **Fonction** |
| Bis-Trimethoxysiloxyethyl Tetramethyldisiloxyethyl Dimethicone (*) | PMN87176 | 25-45 | Polymère |
| Silica Silylate | 68909-20-6 | 5-20 | Charge |
| Disiloxane | 107-46-0 | 30-70 | Solvant |

| **Mélange B':** | | | |
|---|---|---|---|
| **Ingrédient (Nom INCI)** | **N°CAS** | **Teneurs (%)** | **Fonction** |
| Disiloxane | 107-46-0 | 80-99 | Solvant |
| Tetra T Butyl Titanate | - | 1-20 | Catalyseur |

| | | | |
|---|---|---|---|
| (*) Il est à noter que les composés X et Y identiques sont réunis dans le mélange A'. | | | |

Lorsque le polymère filmogène selon l'invention est dispersé dans le solvant organique, la composition selon l'invention comprend avantageusement au moins une dispersion stable de particules de polymère essentiellement sphériques d'un ou plusieurs polymères. Avant leur incorporation dans la composition de l'invention, les particules sont généralement dispersées dans une phase grasse liquide physiologiquement acceptable, telle que les huiles hydrocarbonées ou les huiles siliconées. Selon un mode de mise en oeuvre, ces dispersions sont généralement connues comme « NAD » (dispersions non aqueuses) de polymère par opposition aux réseaux qui sont des dispersions aqueuses de polymère.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1 µm.

Les polymères en dispersion pouvant être utilisés dans la composition de l'invention ont préférablement un poids moléculaire allant d'environ 2000 à 10.000.000 et une Tg allant de -100°C à 300°C et préférablement de -10°C à 80°C.

Parmi les polymères filmogènes en dispersion, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Selon un mode de mise en oeuvre, les particules de polymère sont stabilisées par un stabilisant solide à température ambiante, qui peut être un polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Lorsqu'une dispersion aqueuse de particules polymères est utilisée, la teneur en matière sèche de ladite dispersion aqueuse peut être de l'ordre de 3 à 60% en poids, et préférablement de 10 à 50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

De façon non limitative, des polymères filmogènes préférés sont choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides; polymères ou copolymères acryliques et/ou vinyliques ; copolymères acryliques-silicone ; polyacrylamides ; polymères siliconés comme les polyuréthanes ou acryliques siliconés, polymères fluorés ; celluloses et leurs mélanges.

Les polymères filmogènes hydrophobes selon l'invention peuvent être sélectionnés sur leurs propriétés mécaniques. De telles propriétés peuvent être la flexibilité, la dureté, l'adhésion, lé rémanence, la résistance à l'eau ou à d'autres composés chimiques, la résistance à l'abrasion. Il est aussi possible de prendre avantage des propriétés plus versatiles des polymères blocs (polymères constitués de deux ou plus segments de polymères distincts), des polymères greffés (polymères ayant une chaîne pendante polymérique greffée sur le squelette de l'homopolymère ou du copolymère), des hétéropolymères (polymères comprenant deux ou plus monomères différents). Dans les copolymères par exemple, la quantité de blocs durs et mous ont un impact significatif sur les propriétés du polymère.

De plus il est possible de mélanger deux polymères ou plus pour parvenir à la propriété souhaitée. Des exemples de combinaisons peuvent être polyuréthanes et polyacrylates, polyuréthane et polyesters, deux polymères ayant une partie siliconée, polyuréthane et polymère ayant une partie siliconée.

Selon un mode de réalisation particulier, le polymère filmogène hydrophobe est un polymère non ionique. Selon un autre mode de réalisation, le polymère filmogène est solide à 25°C, en ce sens que l'on n'observe pas son écoulement à l'oeil nu au bout d'une durée d'une heure.

Le polymère peut être présent dans la composition en une teneur allant de 0,1% à 40% en poids, par rapport au poids total de la composition, de préférence allant de 0,1% à 30% en poids, de préférence allant de 0,5 à 20% en poids, préférentiellement allant de 1 % à 20% en poids, et plus préférentiellement allant de 1 % à 15% en poids.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

De préférence, le plastifiant a une masse moléculaire inférieure ou égale à 5000 g/mol, de préférence inférieure ou égale à 2000 g/mol, préférentiellement inférieure ou égale à 1000 g/mol, et plus préférentiellement inférieure ou égale à 900 g/mol. Le plastifiant a avantageusement une masse moléculaire supérieure ou égale à 100 g/mol.

Ainsi, la composition peut comprendre, en outre, au moins un agent plastifiant. En particulier, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000, tels que par exemple
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le dipropylène glycol butyléther. De tels composés sont commercialisés par Dow Chemical sous les dénominations Dowanol PPH et Dowanol DPnB.
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S, en particulier le monoester résultant de la réaction de l'acide isobutyrique et d'octanediol tel que le triméthyl-2,2,4 pentane diol 1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- leurs mélanges.

Plus particulièrement, le plastifiant peut être choisi parmi les esters d'au moins un acide carboxylique comprenant 1 à 7 atomes de carbones et d'un polyol comprenant au moins 4 groupes hydroxyles.

Le polyol selon l'invention peut être un ose - polyhydroxyaldéhyde (aldose) ou polyhydroxycétone (cétose) - cyclisé ou non. Le polyol est de préférence un ose cyclisé sous forme d'hémi-acétal.

Le polyol peut être un mono- ou un polysaccharide comprenant de 1 à 10 oses, de préférence de 1 à 4, de préférence encore un ou deux oses. Le polyol peut être choisi parmi l'érythritol, le xylitol, le sorbitol, le glucose, le saccharose, le lactose, le maltose.

Le polyol selon l'invention est de préférence un disaccharide. Parmi les disaccharides, on peut citer le saccharose (appelé également alpha-D-glucopyranosyl-(1-2)-béta-D-fructofuranose), le lactose (appelé également béta-D-galactopyranosyl-(1-4)-béta-D-glucopyranose) et le maltose (appelé également alpha-D-glucopyranosyl-(1-4)-béta-D-glucopyranose), et de préférence le saccharose.

L'ester selon l'invention peut être constitué d'un polyol estérifié par au moins deux acides monocarboxyliques différents, ou par au moins trois acides monocarboxyliques différents.

L'ester selon l'invention peut être un copolymère de deux esters, en particulier un copolymère i) d'un saccharose substitué par des groupements benzoyle et ii) d'un saccharose substitué par des groupements acétyle et/ou isobutyryle.

L'acide carboxylique est de préférence un acide monocarboxylique comprenant de 1 à 7 atomes de carbones, de préférence de 1 à 5 atomes de carbone, par exemple choisi parmi les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique, l'acide benzoïque, et leurs mélanges, et plus préférentiellement.

Selon un mode de mise en oeuvre préféré, l'ester est le di-acétate hexa-(2-méthylpropanoate) de saccharose, tel que celui vendu sous la dénomination "Sustane SAIB Food Grade Kosher" par la société EASTMAN CHEMICAL.

Selon un autre mode de réalisation, le plastifiant peut être choisi parmi les esters d'acide polycarboxylique aliphatique ou aromatique et d'alcool aliphatique ou aromatique comprenant de 1 à 10 atomes de carbone.

L'alcool aliphatique ou aromatique comprend de 1 à 10 atomes de carbone, de préférence de 1 à 8, par exemple de 1 à 6. Il peut être choisis parmi les alcools R1OH, tels que R1 représente méthyle, éthyle, propyle, isopropyle, butyle, hexyle, éthylhexyle, décyle, isodécyle, benzyle, ou benzyle substitué par un alkyle comprenant 1 à 3 atomes de carbone, et leurs mélanges.

L'acide polycarboxylique aliphatique ou aromatique comprend de préférence de 3 à 12 atomes de carbone, de préférence de 3 à 10 atomes de carbone, de préférence de 3 à 8 atomes de carbone, par exemple 6 ou 8 atomes de carbones.

L'acide polycarboxylique aliphatique ou aromatique est avantageusement choisi parmi les acides dicarboxyliques et les acides tricarboxyliques.

Parmi les acides dicarboxyliques aliphatiques, on peut citer ceux de formule HOOC-(CH₂)ₙ-COOH, dans laquelle n est un entier allant de 1 à 10, de préférence allant de 2 à 8, par exemple égal à 2, 4, 6 ou 8.

On préfère les acides dicarboxyliques choisis parmi l'acide succinique, l'acide adipique et l'acide sébacique.

Parmi les acides dicarboxyliques aromatiques, on peut citer l'acide phtalique.

Parmi les acides tricarboxyliques, on peut citer les triacides qui correspondent à la formule : 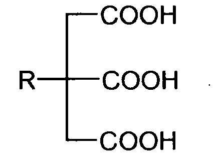 dans laquelle R représente un groupement -H, -OH ou -OCOR' dans lequel R' représente un groupement alkyle ayant de 1 à 6 atomes de carbone. De préférence, R représente un groupement -OCOCH₃.

L'acide tricarboxylique est notamment choisi parmi l'acide acétyl-citrique, l'acide butyroyl-citrique, l'acide citrique.

Parmi les esters d'acide tricarboxylique, on peut utiliser les esters dérivés de l'acide citrique (ou citrates) tels que l'acétyl-citrate de tributyle, l'acétyl-citrate de triéthyle, l'acétyl-citrate de triéthylhexyle, l'acétyl-citrate de trihexyle, le butyroyl- citrate de trihexyle, le citrate de triisodécyle, le citrate de triisopropyle, le citrate de tributyle et le citrate de tri(éthyl-2- hexyle). Comme références commerciales de plastifiants mentionnés ci-dessus on peut citer, la gamme Citroflex commercialisée par Vertellus avec notamment le Citroflex A4 et le Citroflex C2.

Parmi les esters de l'acide adipique, on peut citer l'adipate de dibutyle et l'adipate de di(éthyl-2-hexyle).

Parmi les esters de l'acide sébacique, on peut citer le sébaçate de dibutyle, le sébaçate de di(éthyl-2-hexyle), le sébaçate de diéthyle et le sébaçate de diisopropyle.

Parmi les esters de l'acide succinique, on peut citer le succinate de di(éthyl-2-hexyle) et le succinate de diéthyle.

Parmi les esters de l'acide phtalique, on peut citer le phtalate de butyle et de benzyle, le phtalate de dibutyle, le phtalate de diéthylhexyle, le phtalate de diéthyle et le phtalate de diméthyle.

Avantageusement, le plastifiant peut être présent dans la composition en une teneur telle que le rapport massique entre le polymère filmogène hydrophobe et le plastifiant est compris entre 0,5 et 100, de préférence entre 1 et 50, de préférence entre 1 et 10.

Selon l'invention, la composition appliquée sur les cheveux contient au moins un solvant volatil. Dans le cadre de l'invention, on entend par solvant volatil, un composé liquide à la température ambiante (20°C) et à la pression atmosphérique présentant une pression de vapeur à 20°C supérieure à 0,1 mmHg et de préférence comprise entre 0,1 et 300 mmHg, encore plus préférentiellement entre 0.5 et 200 mmHg.

Ce solvant volatil peut être de l'eau, un solvant organique non siliconé, un solvant organique siliconé ou leurs mélanges. A titre de solvant organique non siliconé volatil, on peut citer :
- les alcanols volatils en C₁-C₄ tels que l'éthanol, l'isopropanol;
- les alcanes volatils en C₅-C₇ tels que le n-pentane, l'hexane, le cyclopentane, le 2,3-diméthylbutane, le 2,2-diméthylbutane, le 2-méthylpentane, le 3-méthylpentane ;
- les esters d'acides en C₁-C₂₀ liquides et d'alcools en C₁-C₈ volatils tels que l'acétate de méthyle, l'acétate de n-butyle, l'acétate d'éthyle, l'acétate de propyle, l'acétate d'isopentyle, le 3-éthoxypopionate d'éthyle;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;
- les éthers volatils tels que le diméthoxyméthane, le diéthoxyéthane, le diéthyléther ;
- les éthers de glycol volatils comme le 2-butoxyéthanol, le butyle diglycol, le monoméhyléther de diéthylène glycol, le n-butyléther de propylène glycol, l'acétate de monométhyl éther de propylène glycol.
- les huiles hydrocarbonées volatiles telles que les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, et par exemple les huiles vendues sous les noms commerciaux d'Isopars ou de Permetyls, et leurs mélanges. On peut aussi citer les neopentanoate d'isohexyle ou d'isodecyle.
- les perfluoroalcanes volatils en C₄-C₁₀ tels que dodecafluoropentane, le tetradecafluorohexane, le decafluoropentane
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine.
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical -(CH₂)ₙ-CH₃ ou -(CF₂)ₘ-CF₃, m étant 2, 3, 4 ou 5.
Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200°C.

Selon un mode de réalisation particulier, le solvant organique non siliconé est choisi parmi l'éthanol, l'isopropanol, l'acétone, l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, le décaméthyltétrasiloxane, et leurs mélanges. Selon un mode de réalisation particulier, le composé siliconé est choisi parmi le cyclopentadiméthylsiloxane et le dodécaméthylcyclohexasiloxane.

Selon un mode de réalisation particulier, le solvant siliconé volatil présente une viscosité inférieure à 50 centistokes.

De préférence, la silicone volatile est cyclique et choisie parmi le décaméthylcyclopentasiloxane, l'octaméthyltrisiloxane et le décaméthyltétrasiloxane.

A titre d'exemple on peut citer la décaméthylcyclopentasiloxane commercialisée sous le nom DC-245 par la société Dow Corning, l'octaméthyltrisiloxane commercialisée sous le nom DC-200 Fluid 1 cst par la société Dow Corning et le décaméthyltétrasiloxane commercialisée sous le nom DC-200 Fluid 1.5 cst par la société Dow Corning

Cette silicone volatile cyclique présente généralement une viscosité faible, par exemple une viscosité inférieure à 5 cSt à 25°C.

De préférence la silicone volatile est cyclique et est le décaméthylcyclopentasiloxane commercialisé sous le nom DC-245 par la société Dow Corning.

Le solvant volatil peut être présent dans la composition utile dans le procédé de l'invention en une teneur allant de 0,1% à 95% en poids, par rapport au poids total de la composition, de préférence allant de 1% à 70% en poids, et préférentiellement allant de 5% à 90% en poids.

La composition de l'invention peut de plus contenir d'autres solvants organiques non volatils tels que :
- les alcools aromatiques non volatils tels que l'alcool benzylique, le phenoxyéthanol ;
- les esters d'acides en C₁-C₂₀ liquides et d'alcools en C₁-C₈ non volatils tels que le myristate d'isopropyle ;
- l'éthylène carbonate, le propylène carbonate; le butylène carbonate ;
- les polyols non volatils tels que le glycérol, l'éthylène glycol, le dipropylène glycol, le butylène glycol,
- les éthers de glycol non volatils comme le monoéthyléther de diéthylène glycol, le mono n-butyl éther de dipropylène glycol.
- les huiles hydrocarbonées non volatiles telles que l'isohexadécane.
- Les alcools gras liquides non volatils en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle
- Les solvants perfluorés non volatils tels que le perfluoroperhydrophenanthrene, vendu sous la dénomination de "Flutec PC11®" par la Société F2 Chemicals

La composition comprend des pigments. Une telle composition permet d'obtenir des gainages colorés et rémanents, et ceci sans dégradation des fibres kératiniques.

Par pigment, on entend tous les pigments apportant de la couleur aux matières kératiniques ou des pigments blancs tels que le dioxyde de titane qui n'apporte que du blanc aux matières kératiniques.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome et le bleu ferrique.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

A titre d'exemple on peut aussi citer les pâtes pigmentaires de pigment organique telles que les produits vendus par la société HOECHST sous le nom :
- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1184426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica recouvert de titane, ou d'oxychlorure de bismuth, les pigments nacrés colorés tels que le mica recouvert de titane et d'oxydes de fer, Ile mica recouvert d'oxyde de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO₂-laque), Prestige commercialisée par Eckart (Mica-TiO₂), Prestige Bronze commercialisée par Eckart (Mica-Fe2O3),Colorona commercialisée par Merck (Mica-TiO₂-Fe2O₃).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C₈ à C₂₀ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et/ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4578266.

De préférence, on utilisera un agent organique lié aux pigments de manière covalente.

L'agent pour le traitement de surface peut représenter de 0,1 à 50% en poids du poids total des pigments traités en surface, de préférence de 0,5 à 30% en poids, et encore plus préférentiellement de 1 à 10% en poids.

De préférence, les traitements en surface des pigments sont choisis parmi les traitements suivants :
- un traitement PEG-Silicone comme le traitement de surface AQ commercialisé par LCW;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone/Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate/Diméthicone et Phosphate de Perfluoalkyle comme le traitement de surface FSA commercialisé par Daito ;
- un traitement Polyméthylhydrogène siloxane/Phosphate de Perfluoroalkyle comme le traitement de surface FS01 commercialisé par Daito ;
- un traitement Lauryl Lysine/Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane/Phosphate de Perfluoroalkyle comme le traitement de surface FOTS commercialisé par Daito ;
- un traitement Copolymère Acrylate/Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

De préférence le pigment est une nacre.

La quantité de pigments peut varier de 0.5% jusqu'à 40% et de préférence de 1 à 20%.

La composition de l'invention peut contenir d'autres espèces colorées ou colorantes telle que des colorants directs hydrophiles ou hydrophobes ou des précurseurs de colorants.

Afin d'obtenir un meilleur étalement de la composition de l'invention ainsi qu'un gainage amélioré, la composition de l'invention peut aussi contenir un ou plusieurs polysiloxanes présentant une viscosité supérieure à 100 cst, préférentiellement supérieure à 300 cst. La viscosité de ces polysiloxanes peut être mesurée selon la norme ASTM D-445. De tels polysiloxanes peuvent être des huiles, des gommes ou des résines de silicone, les silicones réticulées.

A titre de polysiloxanes de viscosité supérieure à 100 cst, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et/ou aromatiques, éventuellement fluorés, ou par des groupements fonctionnels tels que des groupements hydroxyles, thiols et/ou amines.

De tels polysiloxanes peuvent choisis parmi les silicones de formule (I): 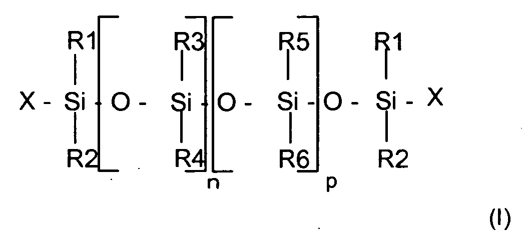 dans laquelle :
R₁, R₂, R₅ et R₆ sont, ensemble ou séparément, un radical alkyle ayant 1 à 6 atomes de carbone, R₃ et R₄ sont, ensemble ou séparément, un radical alkyle ayant de 1 à 6 atomes de carbone, un radical vinyle, un radical aryle, un radical amine, un radical hydroxyle, X est un radical alkyle ayant de 1 à 6 atomes de carbone, un radical hydroxyle, un radical vinyle, un radical amine, n et p étant des entiers choisis de manière à obtenir une viscosité supérieure à 300 cst.

A titre d'exemple, on peut citer les polydiméthylsiloxanes suivantes :
○ les substituants R₁ à R₆ et X représentent un groupement méthyle, comme celle vendue sous la dénomination Baysilicone TP 3898 par la société Général Electric, et celle vendue sous la dénomination AK 500000 par la société Waker,
○ les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning.
○ les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning.
○ les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning.
○ Les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante : 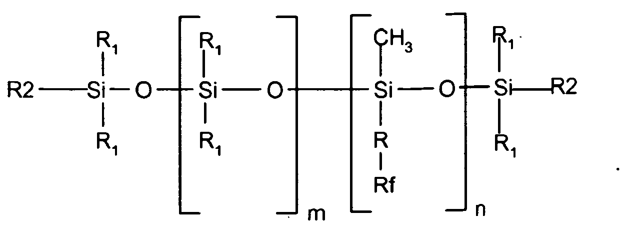 dans laquelle :
R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, R₁ représente, indépendamment l'un de l'autre, un radical alkyle en C₁-C₂₀, un radical hydroxyle, un radical phényle, R₂ représente R₁ ou R_{f}
   - m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être sous forme de résine. Par le terme «résine», on entend une structure tridimensionnelle réticulée ou non. A titre d'exemple de résine de polysiloxane, on peut citer les silsesquioxanes et les siloxysilicates.

Dans un mode de réalisation de l'invention, les polysiloxanes utiles dans la composition de l'invention sont solubles ou dispersables dans la composition de l'invention. Dans un mode de réalisation, la résine de silicone est solide à 25°C.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ces organopolysiloxane peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.

La synthèse de ces organopolysiloxane est décrite dans les brevets suivants :
- US 5266321 de Kobayashi Kose,
- US 4742142 de Toray Silicone,
- US 5654362 de Dow Corning Corp,
- la demande de brevet FR 2 864 784.

Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 µm, de préférence de 3 à 200 µm et mieux de 3 à 50 µm. Ces particules peuvent avoir toute forme et par exemple être sphériques, plates ou amorphes.

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et/ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US4970252, US4987169, US 5412004, US5654362, US5760116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US5236986, US5412004, US5837793, US5811487.

Comme organopolysiloxanes réticulés émulsionnants, on peut utiliser ceux commercialisés sous les dénominations "KSG-21", "KSG-20", "KSG-30", "X-226146" par la société Shin Etsu, et "DC901 0", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US5538793.

De tels élastomères sont vendus sous les dénominations "KSP-100", "KSP-101", "KSP-102", "KSP-103", KSP-104", "KSP-105" par la société Shin Etsu.

Lorsqu'ils sont présents, la quantité de ces composés siliconés est généralement comprise entre 0.1% et 30% en poids, notamment entre 0.1% et 20% en poids et préférentiellement entre 0.1 et 10% en poids.

La composition selon l'invention comprend au moins un agent épaississant choisi parmi les agents épaississants polymériques, les agents épaississants minéraux et leurs mélanges.

L'épaississant peut être minéral ou organique, polymère ou non polymère. L'épaississant peut être choisi pour épaissir une phase aqueuse ou une phase grasse de la composition selon les cas.

On entend par épaississant, un composé qui modifie la rhéologie du milieu dans lequel il est incorporé.

L'épaississant de milieu aqueux peut être choisi parmi :
- les argiles hydrophiles,
- la silice pyrogénée hydrophile.
- les épaississants cellulosiques hydrosolubles, tels que l'hydroxyethylcellulose, la méthylcellulose, l'hydroxypropylcellulose. Parmi celles-ci on peut citer notamment les gommes vendues sous la dénomination Cellolize QP 4400 H par a société Amercol.
- les gommes de guar non ionique comprenant des groupements hydroxyalkyle en C₁-C₆. On peut mentionner à titre d'exemple, les groupements hydroxymethyle, hydroxypropyle et hydroxybutyle. De telles gommes de guar sont notamment vendue sous les dénominations commerciales JAGUAR HP8, JAGUAR HP60, JAGUAR HP120, JAGUAR HP105 par la société MEYHALL ou sous la dénomination GALACTASOL 40H4FD2 par la société AQUALON.
- les carraghénanes
- les gommes de caroube, de scléroglucane, de gellane, de rhamsan, de karaya
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

De tels épaississants sont notamment décrits dans les demandes EP-A-1400234.

L'épaississant de milieu huileux peut être choisi parmi
- les argiles organophiles,
- les silices pyrogénées hydrophobes
- les gommes de guar alkylées (avec groupe alkyle en C1-C6), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619; en particulier les résines de polyamides (notamment comprenant des groupes alkyles ayant de 12 à 22 atomes de carbone) telles que celles décrites dans US-A-5783657.
- les résines de polyamides siliconées telles que décrites dans la demande EP-A-1266647 , dans la demande de brevet français déposée sous le n° 0216039.

De tels épaississants sont notamment décrits dans les demandes EP-A-1400234.

L'épaississant peut être un agent gélifiant organique, c'est-à-dire un agent comprenant au moins un composé organique. Les organogélateurs peuvent être choisis parmi ceux décrits dans la demande WO-A-03/105788.

Plus précisément, l'agent épaississant polymérique présent dans la composition selon l'invention est un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5221534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en C₃-C₄.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène/propylène.

Plus précisément, les argiles organophiles sont des argiles modifiées par des composés chimiques rendant l'argile apte à gonfler.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson".

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

L'agent épaississant est présent dans la composition en une teneur totale allant de 0,1 à 10% en poids par rapport au poids total de la composition, de préférence allant de 0.5 à 7% en poids, et plus préférentiellement allant de 1 à 5% en poids.

Les compositions peuvent également contenir au moins un agent utilisé habituellement en cosmétique, choisi, par exemple, parmi des agents réducteurs, des corps gras, des adoucissants, des agents anti-mousse, des agents hydratants, des filtres UV, des colloïdes minéraux, des peptisants, des solubilisants, des parfums, des tensio-actifs anioniques, cationiques, non ioniques ou amphotères, des protéines, des vitamines, des propulseurs, les cires oxyéthylénées ou non, les paraffines, les acides gras en C₁₀-C₃₀ tels que l'acide stéarique, l'acide laurique, les amides gras en C₁₀-C₃₀ tel que le diéthanolamide laurique.

Les additifs ci dessus sont en général présents en quantité comprise pour chacun d'eux entre 0,01 et 20% en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte. La composition peut également se présenter sous forme de laque.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition peut être une composition anhydre, c'est-à-dire une composition contenant moins de 2% en poids d'eau, voire moins de 0,5% d'eau, notamment exempte d'eau, l'eau n'étant pas ajoutée lors de la préparation de la composition mais correspondant à l'eau résiduelle apportée par les ingrédients mélangés.

La composition décrite ci-dessus peut être mise en oeuvre sur cheveux secs ou humides ainsi que sur tous types de cheveux clairs ou foncés, naturels ou colorés, permanentés, décolorés ou défrisés.

Selon un mode de réalisation particulier du procédé de l'invention, les cheveux sont lavés avant application de la composition décrite ci-dessus.

L'application sur les cheveux peut être mise en oeuvre par exemple au moyen d'un peigne, d'un pinceau à l'aide d'une brosse ou aux doigts.

L'application de la composition est ensuite suivie d'un séchage à une température supérieure à 40°C. Selon un mode de réalisation particulier, cette température est supérieure à 45°C. Selon un mode de réalisation particulier, cette température est supérieure à 45°C et inférieure à 220°C.

Le séchage peut être réalisé immédiatement après l'application ou après un temps de pose pouvant aller de 1 minute à 30 minutes.

De préférence les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser, un climazon...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

### EXEMPLES

### EXEMPLE 1 (hors invention)

La composition suivante est réalisée :

| | | |
|---|---|---|
| Acéto-propionate de cellulose commercialisée sous la référence CAB-381-0.5 par Eastman Chemical | 10g | |
| Nacre Mica-Oxyde de fer commercialisée sous la référence Prestige Soft Bronze par Eckart | | 10g |
| Acétate d'éthyle | | 80g |

0.5g de la composition est appliqué sur une mèche de 1g de cheveux propres et secs. La mèche est immédiatement séchée au sèche-cheveux à une température de 80°C pendant 1 minute en appliquant un peignage constant durant le séchage. On obtient une mèche colorée dont les cheveux sont individualisés et dont la couleur est rémanente au shampooing.

### EXEMPLE 2

La composition suivante est réalisée :

| | | |
|---|---|---|
| Copolymère d'isobutylmethacrylate / bis-hydroxypropyl dimethicone acrylate à 40% dans l'isododécane commercialisé par Grant Industrie sous le nom Granacrysil BMAS | 25g | |
| Nacre Mica-Oxyde de fer | | |
| commercialisée sous la référence Prestige Soft Bronze par Eckart | | 10g |
| Poly diméthyl siloxane alpha-omega dihydroxylé / cyclopentadiméthylsiloxane (14,7 / 85,3) commercialisé par Dow Corning sous le nom de DC 1501 Fluid | | 50g |
| Isododécane | 15g | |

0.5g de la composition est appliqué sur une mèche de 1g de cheveux propres et secs. Après 2 minutes de pause, la mèche est séchée au sèche-cheveux à une température de 80°C pendant 2 minutes. On obtient une mèche colorée dont les cheveux sont individualisés et dont la couleur est rémanente au shampooing.

### EXEMPLE 3

La composition suivante est réalisée :

| | | |
|---|---|---|
| Copolymere acide acrylique / methacrylate d'isobutyle / methacrylate d'ethyle / n-tertio-octylacrylamide, commercialisé sous la référence DERMACRYL® LT par la société NATIONAL STARCH | 10g | |
| Nacre Mica-Oxyde de fer commercialisée sous la référence Prestige Soft Bronze par Eckart | | 10g |
| Poly diméthyl siloxane alpha-omega dihydroxylé / cyclopentadiméthylsiloxane (14,7 / 85,3) commercialisé par Dow Corning sous le nom de DC 1501 Fluid | | 40g |
| Ethanol | | 40g |

0.5g de la composition est appliqué sur une mèche de 1g de cheveux propres et secs. Après 2 minutes de pause, la mèche est séchée au sèche-cheveux à une température de 80°C pendant 2 minutes en brushant la mèche durant le séchage. On obtient une mèche colorée dont les cheveux sont individualisés et dont la couleur est rémanente au shampooing.

### EXEMPLE 4

La composition suivante est réalisée :

| | |
|---|---|
| DC245 Fluid commercialisée par Dow Corning | 65g |
| DC1501 Fluid commercialisé par Dow Corning | 20g |
| Nacre mica enrobé d'oxyde de fer brun commercialisée par Eckart sous le nom Prestige Bronze | 10g |
| BioPSA 7-4400 commercialisé par Dow Corning | 5g |

0.5g de la composition est appliqué sur une mèche de 1g de cheveux propres et humides. Après 2 minutes de pause, la mèche est séchée à l'aide d'un fer à lisser à

### 180°C. On obtient une mèche colorée dont les cheveux sont individualisés et dont la couleur est rémanente au shampooing.

### EXEMPLE 5

La composition suivante est réalisée :

| | |
|---|---|
| Isopropanol | 45g |
| Cyclopentadiméthylsiloxane commercialisé par Dow Corning sous le nom de DC245 Fluid | 30g |
| Polydiméthylsiloxane commercialisée par Dow Corning sous la référence Dow Corning 200 Fluid 60000 cs | 5g |
| Nacre mica enrobé d'oxyde de fer brun commercialisée par Eckart sous le nom Prestige Bronze | 10g |
| Copolymère diméthylpolysiloxane/urée commercialisé sous la référence Wacker-Belsil□ UD 60 par Wacker | 10g |

0.5g de la composition est appliqué sur une mèche de 1g de cheveux propre et secs. Sans temps de pause, la mèche est séchée au sèche-cheveux à 80°C avec le débit d'air maximum en brushant la mèche durant le séchage pendant 2 minutes. On obtient une mèche colorée dont les cheveux sont individualisés et dont la couleur est rémanente au shampooing.

### EXEMPLE 6

La composition suivante est réalisée :

| | |
|---|---|
| Nacre mica enrobé d'oxyde de fer brun commercialisée par Eckart sous le nom Prestige Bronze | 10g |
| Polymethylsilsesquioxane commercialisé sous la référence Wacker Belsil PMS MK Powder par Wacker | 10g |
| Cyclopentadiméthylsiloxane commercialisé par Dow Corning sous le nom de DC245 Fluid | 80g |

0.5g de la composition est appliqué sur une mèche de 1g de cheveux propre et humides. Sans temps de pause, la mèche est séchée au sèche-cheveux à 80°C avec le débit d'air maximum en brushant la mèche durant le séchage pendant 2 minutes. On obtient une mèche colorée dont les cheveux sont individualisés et dont la couleur est rémanente au shampooing.

### EXEMPLE 7

La composition suivante est réalisée :

| | | |
|---|---|---|
| Nacre mica enrobé d'oxyde de fer brun commercialisée par Eckart sous le nom Prestige Bronze | 10g | |
| Copolymères acrylates/C₁₂-₂₂ alkylméthacrylate en dispersion à 48% dans l'eau commercialisé par Rohm and Haas sous la référence SOLTEX OPT | | 20g |
| 7-3100 Gum Blend HIP Emulsion commercialisée par Dow Corning | 20g | |
| Eau | 50g | |

0.5g de la composition est appliqué sur une mèche de 1g de cheveux propre et humides. Sans temps de pause, la mèche est séchée au sèche-cheveux à 80°C avec le débit d'air maximum en brushant la mèche durant le séchage pendant 2 minutes. On obtient une mèche colorée dont les cheveux sont individualisés et dont la couleur est rémanente au shampooing.

## Revendications

1. Procédé de coloration des cheveux comprenant l'application sur les cheveux d'une composition comprenant un ou plusieurs polymères filmogènes hydrophobes, choisi parmi les polymères ou les copolymères à base de polyuréthane, de polyacrylate, les copolymères d'ester vinyliques, les résines de silicones, les silicones polyurée/polyuréthane, les copolymères à base de résine de silicone et de diméthiconol un ou plusieurs pigments et un solvant volatil, suivi d'une étape de chauffage des cheveux recouverts de la composition, à une température supérieure à 40°C

2. Procédé selon la revendication 1 dans lequel l'étape de chauffage est mise en oeuvre à une température supérieure à 45°C.

3. Procédé selon la revendication 1 ou 2 dans lequel l'étape de chauffage est mise en oeuvre entre 45 et 200°C, de préférence entre 45 et 200°C.

4. Procédé selon l'une quelconque des revendications précédentes dans lequel le solvant volatil est l'eau ou un solvant organique choisi parmi l'éthanol, l'isopropanol, l'acétone, l'isododécane, décaméthylcyclopentasiloxane, l'octaméthyltrisiloxane et le décaméthyltétrasiloxane ou leurs mélanges.

5. Procédé selon l'une quelconque des revendications dans lequel la composition comprend de 0,1% à 40% en poids, par rapport au poids total de la composition du polymère filmogène hydrophobe, de préférence allant de 0,1% à 30% en poids, de préférence allant de 0,5 à 20% en poids, préférentiellement allant de 1% à 20% en poids, et plus préférentiellement allant de 1% à 15% en poids.

6. Procédé selon l'une quiconque des revendications précédentes dans lequel la quantité de solvant volatil est comprise entre 0,1 et 95 % en poids du poids total de la composition.

7. Procédé selon l'une quelconque des revendications précédentes dans lequel la quantité de pigment est comprise entre 0.5 et 40%, en poids du poids total de la composition.

8. Procédé selon l'une quelconque des revendications précédentes dans lequel le pigment est une nacre.

9. Procédé selon l'une quelconque des revendications précédentes dans lequel la composition comprend de plus un ou plusieurs polysiloxanes présentant une viscosité supérieure 300 cst.

10. Procédé selon l'une quelconque des revendications précédentes dans lequel l'étape de chauffage est mise en oeuvre avec un flux d'air permettant d'obtenir sur les cheveux une température supérieure à 40°C.

## Patentansprüche

1. Verfahren zum Färben der Haare, bei dem man auf die Haare eine Zusammensetzung, die ein oder mehrere hydrophobe filmbildende Polymere, ausgewählt aus Polymeren oder Copolymeren auf Basis von Polyurethan, Polyacrylat, Vinylester-Copolymeren, Silikonharzen, Polyharnstoff/Polyurethan-Silikonen, Copolymeren auf Basis von Silikonharz und Dimethicon, ein oder mehrere Pigmente und ein flüchtiges Lösungsmittel umfasst, aufbringt und dann die mit der Zusammensetzung bedeckten Haare auf eine Temperatur von mehr als 40°C erhitzt.

2. Verfahren nach Anspruch 1, bei dem man das Erhitzen bei einer Temperatur von mehr als 45°C durchführt.

3. Verfahren nach Anspruch 1 oder 2, bei dem man das Erhitzen zwischen 45 und 200°C und vorzugsweise zwischen 45 und 200°C durchführt.

4. Verfahren nach einem der vorhergehenden Ansprüche, bei dem es sich bei dem flüchtigen Lösungsmittel um Wasser oder ein organisches Lösungsmittel, ausgewählt aus Ethanol, Isopropanol, Aceton, Isododecan, Decamethylcyclopentasiloxan, Octamethyltrisiloxan und Decamethyltetrasiloxan, oder Mischungen davon handelt.

5. Verfahren nach einem der vorhergehenden Ansprüche, bei dem die Zusammensetzung 0,1 bis 40 Gew.-%, bezogen auf das Gesamtgewicht der Zusammensetzung, hydrophobes filmbildendes Polymer, vorzugsweise im Bereich von 0,1 bis 30 Gew.-%, vorzugsweise im Bereich von 0,5 bis 20 Gew.-%, bevorzugt im Bereich von 1 bis 20 Gew.-% und weiter bevorzugt im Bereich von 1 bis 15 Gew.-%, umfasst.

6. Verfahren nach einem der vorhergehenden Ansprüche, bei dem die Menge an flüchtigem Lösungsmittel zwischen 0,1 und 95 Gew.-% des Gesamtgewichts der Zusammensetzung liegt.

7. Verfahren nach einem der vorhergehenden Ansprüche, bei dem die Menge an Pigment zwischen 0,5 und 40 Gew.-% des Gesamtgewichts der Zusammensetzung liegt.

8. Verfahren nach einem der vorhergehenden Ansprüche, bei dem es sich bei dem Pigment um einen Perlmutt handelt.

9. Verfahren nach einem der vorhergehenden Ansprüche, bei dem die Zusammensetzung außerdem ein oder mehrere Polysiloxane mit einer Viskosität von mehr als 300 cSt umfasst.

10. Verfahren nach einem der vorhergehenden Ansprüche, bei dem man das Erhitzen mit einem Luftstrom durchführt, mit dem auf den Haaren eine Temperatur von mehr als 40°C erhalten werden kann.

## Claims

1. Process for dyeing the hair, comprising the application to the hair of a composition comprising one or more hydrophobic film-forming polymers chosen from polymers or copolymers based on polyurethane, polyacrylate, vinyl ester copolymers, silicone resins, polyurea/polyurethane silicones, and copolymers based on silicone resin and on dimethiconol, one or more pigments and a volatile solvent, followed by a step of heating the hair covered with the composition to a temperature above 40°C.

2. Process according to Claim 1, in which the heating step is performed at a temperature above 45°C.

3. Process according to Claim 1 or 2, in which the heating step is performed between 45 and 200°C and preferably between 45 and 200°C.

4. Process according to any one of the preceding claims, in which the volatile solvent is water or an organic solvent chosen from ethanol, isopropanol, acetone, isododecane, decamethylcyclopentasiloxane, octamethyltrisiloxane and decamethyltetrasiloxane, or mixtures thereof.

5. Process according to any one of the preceding claims, in which the composition comprises from 0.1% to 40% by weight, preferably ranging from 0.1% to 30% by weight, preferably ranging from 0.5% to 20% by weight, preferentially ranging from 1% to 20% by weight and more preferentially ranging from 1% to 15% by weight, relative to the total weight of the composition, of hydrophobic film-forming polymer.

6. Process according to any one of the preceding claims, in which the amount of volatile solvent is between 0.1% and 95% by weight relative to the total weight of the composition.

7. Process according to any one of the preceding claims, in which the amount of pigment is between 0.5% and 40% by weight relative to the total weight of the composition.

8. Process according to any one of the preceding claims, in which the pigment is a nacre.

9. Process according to any one of the preceding claims, in which the composition also comprises one or more polysiloxanes with a viscosity of greater than 300 cSt.

10. Process according to any one of the preceding claims, in which the heating step is performed with a flow of air that makes it possible to obtain on the hair a temperature above 40°C.
